# EUROPEAN PATENT SPECIFICATION

(11) **EP 2 663 283 B1**
(45) Date of publication and mention of the grant of the patent: **10.05.2017**
(21) Application number: 12702349.7
(22) Date of filing: 13.01.2012
(51) Int. Cl.: A61K 9/00, A61K 9/20, A61K 9/28, A61K 31/53, A61K 47/02, A61K 47/12, A61K 47/26, A61K 47/32, A61K 47/36, A61K 47/38, A61P 35/00

(54) **HIGH DRUG LOAD TABLET FORMULATION OF BRIVANIB ALANINATE**
TABLETTE MIT HOHEM WIRKSTOFFGEHALT AUS BRIVANIB-ALANINAT
COMPRIMÉ CONTENANT UNE CHARGE ÉLEVÉE DE BRIVANIB ALANINATE

(30) Priority: 14.01.2011 US 201161432801 P
(43) Date of publication of application: 20.11.2013
(73) Proprietor: Bristol-Myers Squibb Company, Princeton, NJ 08543 (US)
(72) Inventor: NARANG, Ajit S., NJ 08903 (US); BADAWY, Sherif Ibrahim Farag, NJ 08903 (US); SUBRAMANIAN, Ganeshkumar A., NJ 08903 (US); LAMARCHE, Keirnan Ryan, NJ 08903 (US); BINDRA, Dilbir S., NJ 08903 (US); RAO, Venkatramana M., NJ 08903 (US)
(74) Representative: Mena, Sandra
(86) International application number: PCT/US2012/021199
(87) International publication number: WO 2012/097222

(56) References cited:
- WO-A1-2007/038648
- T. MEKHAIL ET AL: "Metabolism, Excretion, and Pharmacokinetics of Oral Brivanib in Patients with Advanced or Metastatic Solid Tumors", DRUG METABOLISM AND DISPOSITION, vol. 38, no. 11, 29 July 2010 (2010-07-29) , pages 1962-1966, XP55025143, ISSN: 0090-9556, DOI: 10.1124/dmd.110.033951
- Bristol-Myers Squibb: "MAD in Cancer Patients: Safety of BMS-582664 in Patients With Advanced or metastatic Solid Tumors", US National Institute of Health , 3 November 2008 (2008-11-03), XP002674371, Retrieved from the Internet: URL:http://clinicaltrials.gov/ct2/show/stu dy/NCT00207103 [retrieved on 2012-04-20]
- HUYNH H ET AL: "Brivanib alaninate: VEGFR/FGFR inhibitor oncolytic", DRUGS OF THE FUTURE 2009 PROUS SCIENCE ESP LNKD- DOI:10.1358/DOF.2009.034.11.1436615, vol. 34, no. 11, November 2009 (2009-11), pages 881-895, XP002674372, ISSN: 0377-8282
- ZHEN-WEI CAI ET AL: "Discovery of Brivanib Alaninate (( S )-(( R )-1-(4-(4-Fluoro-2-methyl-1 H -indol-5-yloxy)-5-methylpyrrolo[2,1- f ][1,2,4]triazin-6-yloxy)propan-2-yl)2-amin opropanoate), A Novel Prodrug of Dual Vascular Endothelial Growth Factor Receptor-2 and Fibroblast Growth Factor Receptor-1 Kinase Inhibitor (BMS", JOURNAL OF MEDICINAL CHEMISTRY, vol. 51, no. 6, 1 March 2008 (2008-03-01), pages 1976-1980, XP55025169, ISSN: 0022-2623, DOI: 10.1021/jm7013309
- AJIT S. NARANG ET AL: "Reversible and pH-dependent weak drug-excipient binding does not affect oral bioavailability of high dose drugs", JOURNAL OF PHARMACY AND PHARMACOLOGY, vol. 64, no. 4, 13 January 2012 (2012-01-13), pages 553-565, XP55025141, ISSN: 0022-3573, DOI: 10.1111/j.2042-7158.2011.01435.x

## Description

### FIELD OF THE INVENTION

The invention relates to a high drug load tablet formulation of [(1*R*), 2*S*]-2-aminopropionic acid 2-[4-(4-fluoro-2-methyl-1H-indol-5-yloxy)-5-methylpyrrolo[2,1-f][1,2,4]triazin-6-yloxy]-1-methylethyl ester, and to methods of using the formulation in the treatment of cancer.

### BACKGROUND OF THE INVENTION

The compound of formula (I), Brivanib, (R)-1-(4-(4-fluoro-2-methyl-1H-indol-5-yloxy)-5-methylpyrrolo[2,1-f][1,2,4]triazin-6-yloxy)propan-2-ol, is a selective inhibitor of vascular endothelial growth factor (VEGF) and fibroblast growth factor (FGF) receptors, and is useful in the treatment of various forms of cancer.

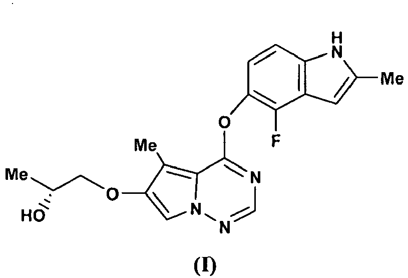

The L-alanine ester prodrug of Compound I (Compound Ia), known as brivanib alaninate, [(1*R*), 2*S*]-2-aminopropionic acid 2-[4-(4-fluoro-2-methyl-1H-indol-5-yloxy)-5-methylpyrrolo[2,1-f][1,2,4]triazin-6-yloxy]-1-methylethyl ester, 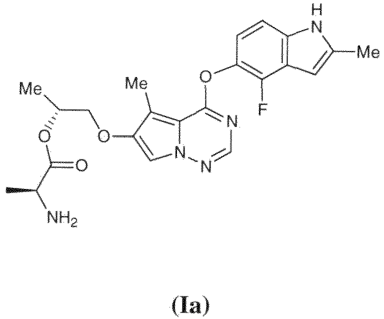 is currently in phase III clinical development as an agent for the treatment of cancer.

The compounds of formula (I) and (Ia), a crystalline form of the compound of formula (Ia) and their preparation have been previously described in U.S. Patent No. 6,869,952, issued March 22, 2005, U.S. Patent No. 7,265, 113, issued September 4, 2007, U.S. Patent No.7,521,450, issued April 21, 2009, U.S. Patent No. 7,671,199 which issued March 2, 2010 and U.S. Patent No.7,932,383 which issued April 26, 2011.

Clinical studies of brivanib alaninate indicated the need for a high dose (200 mg - 800 mg) (T. Mekhail et al., Drugr Metabolism and Disposition, 2010, Vol. 38, no. 11, pp. 1962 - 1966; Bristol-Myers Squibb, "MAD in Cancer Patients: Safety of BSM-S82664 in Patients with Advanced or Metastatic Solid Tumors", 2008, US National Institute of Health). For convenient oral administration, generally the tablet weight should not exceed 1000 mg. Thus, brivanib alaninate tablets require a drug loading of 20% w/w to 80% w/w in the formulation for 200 mg to 800 mg dose per tablet. As the number of tablets required per dose can lead to compliance issues, one objective was to find a formulation that could be commercially made that resulted in less tablets required per dose with the resultant expectation that there would be improved patient compliance.

The overall physical properties and manufacturability of low drug loading formulations is determined predominantly by the inactive ingredients or excipients in the formulation. At high drug loading, the contribution of physical properties of the API to the manufacturability of a formulation becomes predominant.

Brivanib alaninate is an ester compound that is formulated as a free base. It has a pKa of 6.9 and shows pH dependent solubility with limited solubility at neutral and basic pH, but has increasing solubility at acidic pH. However, it also degrades rapidly at acidic pH and at high pH.

Brivanib alaninate solid drug substance shows acceptable stability when stored at room temperature protected from moisture. Its main degradation pathway at neutral and basic pH is hydrolysis to the parent compound, brivanib. Under acidic conditions, it can also undergo diaryl ether cleavage in solution as shown below.

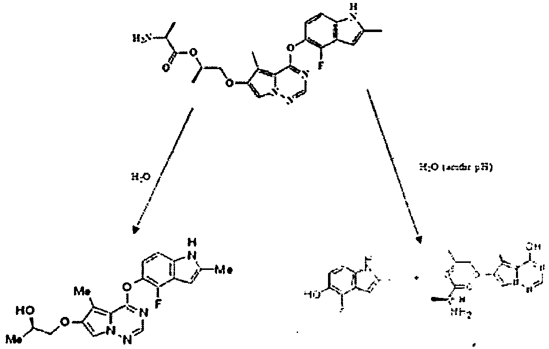

Hydrolytic sensitivity suggests that a solid dosage form, such as a tablet or capsule would be more stable than an aqueous solution or suspension. In addition, a manufacturing process that does not involve the use of water as a processing aid, such as roller compaction, should be preferred over a process that uses water, such as high shear wet granulation or fluid bed granulation.

Brivanib alaninate exhibits sticking to tablet press tools during compression. This problem increases with increasing drug load and compromises its manufacturability above 25% w/w drug load in a roller compaction formulation.

Methods known to reduce the sticking tendency of a drug during tablet compression include reduction of'fines' (powder with particle size below a certain particle diameter), increasing the hardness of tablets, or increasing the concentration of the lubricant, magnesium stearate. Fines in powders are conventionally defined as particles below #100 mesh (149µm), #200 mesh (74µm), or #325 mesh (44µm) by nested sieve analysis - depending on the investigator. For example, one investigator defined fines as particles below 45µm, while another defined fines as particles below 145µm. In any case, this is the only method in the current state-of-the-art to characterize fines in pharmaceutical granules.

These measures, however, are usually effective in overcoming 'borderline cases' of sticking. For example, when using the roller compaction process, at low (25% w/w) drug concentration, the tendency of brivanib alaninate to stick to tablet compression tools was observed. This tendency was highly sensitive to the quantity of fines and the lubricant concentration in the formulation. Therefore, changing the lubricant concentration and the quantity of fines was used to overcome the sticking tendency. However, as the drug load is increased beyond 25% w/w in the formulation, the sticking tendency is significant and is not easily overcome by these measures in a manner that maintains commercial manufacturability of the formulation. For example, if sticking tendency was to be overcome by using excessive quantity of magnesium stearate in the formulation, it would compromise granule bonding during compaction.

Brivanib alaninate shows significant sticking during tableting, which has a direct correlation with the amount of fines (reducing the fines reduces sticking tendency) and an inverse correlation with the amount of lubricant (increasing lubricant concentration reduces sticking tendency).

However, it was unexpected and surprising that for the high drug load formulation of brivanib alaninate, the use of a wet granulation process results in significantly lower sticking tendency than the roller compaction process for the same amount of fines.

The reason for lower sticking tendency of the wet granulated formulation over the roller compacted formulation is attributed to two factors. First, the granulation process modifies exposed particle surface by causing the agglomeration of particles. Thus, the presence of greater proportion of primary drug particles in the formulation is expected to cause sticking, whereas the presence of agglomerated drug particles would not. 'Fines' in wet granulated formulation are agglomerates of primary drug particles, whereas 'fines' in roller compacted samples are largely unagglomerated primary drug particles. Second, is the mechanism of sticking for brivanib alaninate, which is attributed to the interaction of the surface of drug particles with metal surface during tableting. Our studies indicate that the polar functional groups on the exposed surface of the brivanib alaninate crystals are responsible for sticking of drug to the tablet tooling steel surface. These polar functional groups are also expected to be involved in hydrogen bonding by interaction with binder during wet granulation. The polar hydrophilic surface of the binder is expected to bind to the electronegative atoms on the drug's surface. Therefore, particle agglomeration by the wet granulation process would minimize the surface exposure of polar functional groups in brivanib alaninate crystals. This, however, may not be the case with the roller compaction process which does not utilize a binder and a solvent in the manufacturing process.

The use of wet granulation process for brivanib alaninate was not expected to be suitable because brivanib alaninate degrades by hydrolysis. Therefore, any person skilled in the art of pharmaceutical formulation would logically implement all measures to minimize the exposure of drug to water during handling, formulation, processing, and storage. It was surprisingly found that the use of wet granulation for brivanib alaninate did not really result in significant degradation of the drug and that this process was commercially suitable.

### SUMMARY OF THE INVENTION

The present invention is directed to a high drug load tablet formulation of the compound of formula (Ia) and to methods of treating cancer using said formulation.

### DETAILED DESCRIPTION OF THE INVENTION

The present invention is directed to a high drug load tablet formulation of the compound of formula (Ia) 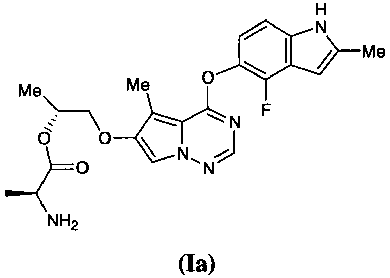 which comprises:
- compound Ia as the active pharmaceutical ingredient in the range of 30 - 80 % w:w,
- one or more fillers in the range of 15 - 65 % w/w,
- binders in the range of 1-20 % w/w.
- desintegrants in the range of 1 - 20 % w/w, and
- lubricants in the range of 0.25 - 2.5% w/w,
wherein the said formulation is obtainable by a wet granulation process.

In another embodiment of the invention, there is disclosed the formulation wherein the active pharmaceutical ingredient is included in the range of 40-70% w/w, one or more filler is included in the range of 25-55% w/w, the binder is included in the range of 2-12%; w/w, one or more disintegrant is included in the range of 2-12% w/w, and the lubricant is included in the range of 0.75-1.5% w/w.

In another embodiment of the invention, there is disclosed the formulation wherein the active pharmaceutical ingredient is included in about 50% w/w, one or more filler is included in about 24.5% w/w, the binder is included in about 3% w/w, one or more disintegrant is included in about 3% w/w, the glidant is included in about 0.5% w/w and the lubricant is included in about 1.0% w/w.

In another embodiment of the invention, there is disclosed the formulation wherein the fillers and disintegrants are intragranular and extragranular.

In another embodiment of the invention, there is disclosed the formulation wherein the fillers are selected from lactose monohydrate and microcrystalline cellulose.

In another embodiment of the invention, there is disclosed the formulation wherein the binder is hydroxypropyl cellulose (HPC), polyvinyl pyrrolidone (PVP), starch or hydroxypropyl methylcellulose (HPMC).

In another embodiment of the invention, there is disclosed the formulation wherein the disintegrants are selected from croscarmellose sodium, crospovidone, starch and sodium starch glycolate.

In another embodiment of the invention, there is disclosed the formulation wherein the lubricant is magnesium stearate.

In another embodiment of the invention, there is disclosed the formulation wherein the active pharmaceutical ingredient is brivanib alaninate, the fillers are microcrystalline cellulose, the binder is hydroxypropyl cellulose, the disintegrants are croscarmellose sodium or crospovidone, the glidant is colloidal silicon dioxide and the lubricant is magnesium stearate.

In another embodiment of the invention, there is disclosed a wet granulated tablet formulation of Compound Ia of the formula 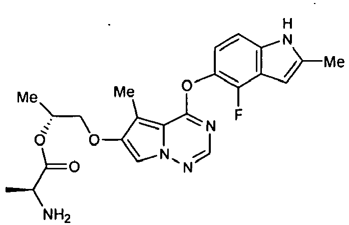 which comprises the active pharmaceutical ingredient and one or more filler, binder, disintegrant, glidant and/or lubricant.

In another embodiment of the invention, there is disclosed the wet granulated tablet formulation wherein the active pharmaceutical ingredient is included in about 50% w/w, one or more filler is included in about 24.5% w/w, the binder is included in about 3% w/w, one or more disintegrant is included in 3% w/w, the glidant is included in about 0.5% w/w and the lubricant is included in about 1.0% w/w.

In another embodiment of the invention, there is disclosed a method of treating cancer, comprising the step of administering to a subject in need thereof an effective amount of the high drug load tablet formulation wherein the cancer is selected from breast cancer, colorectal cancer, hepatocellular carcinoma, non-small cell lung cancer and prostate cancer.

In another embodiment of the invention, there is disclosed a method of treating cancer, comprising the step of administering to a subject in need thereof an effective amount of the wet granulated tablet formulation wherein the cancer is selected from breast cancer, colorectal cancer, hepatocellular carcinoma, non-small cell lung cancer and prostate cancer.

In another embodiment, there is provided a method for treating cancer, comprising administering to a patient in need thereof a therapeutically effective amount of a formulation of the present invention.

In another embodiment, there is provided a method for preparing the formulation of the invention using a wet granulation process.

In another embodiment, there is provided the use of the formulation of the present invention in therapy.

In another embodiment, there is provided the use of the formulation of the present invention in the preparation of a medicament for the treatment of cancer.

The invention may be embodied in other specific forms without departing from the spirit or essential attributes thereof. This invention also encompasses all combinations of preferred aspects and examples of the invention noted herein. It is understood that any and all embodiments of the present invention may be taken in conjunction with any other embodiment to describe additional even more preferred embodiments of the present invention. Furthermore, any elements of an embodiment are meant to be combined with any and all other elements from any of the embodiments to describe additional embodiments.

### DEFINITIONS

"Therapeutically effective amount" is intended to include an amount of a compound of the present invention alone or an amount of the combination of compounds claimed or an amount of a compound of the present invention in combination with other active ingredients effective to inhibit protein tyrosine kinase activity, such as but not limited to VEGF kinase activity, or effective to treat or prevent cancer.

As used herein, "treating" or "treatment" cover the treatment of a disease-state in a mammal, particularly in a human, and include: (a) preventing the disease-state from occurring in a mammal, in particular, when such mammal is predisposed to the disease-state but has not yet been diagnosed as having it; (b) inhibiting the disease-state, i.e., arresting it development; and/or (c) relieving the disease-state, i.e., causing regression of the disease state.

As used herein, the term "filler" refers to any pharmaceutically acceptable inert material or composition added to a formulation to add bulk. Suitable filler include for example, lactose monohydrate and microcrystalline cellulose.

As used herein, the term "disintegrant" refers to materials added to the composition to help it break apart and release the medicaments. Examples of disintegrants include, but are not limited to, non-saccharide water soluble polymers, such as cross-linked povidone. Other disintegrants that can be used include, for example, croscarmellose sodium, starch and sodium starch glycolate.

As used herein, the term "lubricant" refers to any pharmaceutically acceptable agent which reduces surface friction, lubricates the surface of the granule, and decreases the tendency to build up static electricity. Lubricants can also play a related role in improving the compression process by reducing the tendency of the material to adhere to the surface of compression tools. Thus, lubricants can serve as anti-adherents. Examples of suitable lubricants are magnesium stearate, stearic acid or other hydrogenated vegetable oil or triglycerides.

As used herein, the term "binder" refers to any pharmaceutically acceptable compound or composition that can help bind primary powder particles into agglomerates. Examples of suitable binding agents include, but are not limited to, hydroxypropyl cellulose (HPC), polyvinyl pyrrolidone (PVP), starch or hydroxypropyl methylcellulose (HPMC).

### EXAMPLES

The invention is further understood by reference to the following examples, which are intended to be purely exemplary of the invention. The present invention is not limited in scope by the exemplified embodiments, which are intended as illustrations of single aspects of the invention only. Any methods that are functionally equivalent are within the scope of the invention. Various modifications of the invention in addition to those described herein will become apparent to those skilled in the art. Such modifications fall within the scope of the appended claims.

The manufacturing process of this formulation generally involves mixing the drug with dry powder excipients, such as with one or more binder, disintegrant, and filler. Water is then added to this premix while it is being continuously mixed in a mixer such as a high shear granulator. Alternatively, the binder may not be added as a dry powder to the premix, but dissolved in water. Addition of water with continuous mixing leads to the formation of granules, or granulation. The granules are then dried in dry, hot air using equipment and processes such as fluid bed drying or tray drying. Once dried, the granules may be milled using equipment such as comil or Fitzmil to reduce and/or reduce the width of distribution of particle size of granules. The granules are then mixed with extra-granular ingredients, which may include one or more of disintegrant, filler, glidant, and lubricant. The final blend is then compressed on a tablet press into core tablets. The core tablets may optionally be coated with a nonfunctional film coat.

### Example 1: Wet granulation

Three wet granulation formulations (as shown below in Table 1) were manufactured using 75% Compound Ia and intra-granular ingredients (microcrystalline cellulose, binder, and disintegrant). After granulation and drying, the granules were characterized for particle size distribution. Dried granules were lubricated with 1% magnesium stearate and compressed into tablets.

**Table 1: Particle size distribution of granules prepared by wet granulation**

| **Composition** | **Mesh Size** | **100** | **200** |
|---|---|---|---|
| 75% API; 14% Avicel PH 101; 5% Povidone K-30 (dissolved); 5% Croscarmellose Sodium | % w/w granules below | 47 | 31 |
| 75% API; 15.5% Avicel PH 101; 3.5% HPC EX-F (Dry); 5% Crospovidone | % w/w granules below | 27 | 8 |
| 75% API; 9% Avicel PH 101; 10% Starch 1500 (Dry); 5% Crospovidone | % w/w granules below | 10 | 5 |

No sticking was observed in all formulations, irrespective of the content of fines

### Example 2: Roller compaction

Three roller compaction formulations were manufactured using 75% of Compound la and intra-granular ingredients (microcrystalline cellulose, disintegrant, and either of talc, calcium silicate, or hydrogenated vegetable oil, or magnesium stearate). All batches exhibited significant sticking and were not useful for manufacture.

### Example 3: Wet granulation

The typical and preferred ranges of excipients in a typical wet granulation formulation of brivanib alaninate tablets are listed in Table 2. Optionally, the brivanib alaninate tablets could be coated with a non-functional, fast-disintegrating film coat. A tablet formulation of brivanib alaninate was manufactured using the composition listed in Table 3. No sticking was observed.

**Table 2. A typical composition of an immediate release tablet formulation.**

| **S. No.** | **Location in tablet** | **Functional class** | **Examples of components** | **Usable Range*** | **Preferred range*** |
|---|---|---|---|---|---|
| 1 | Intra-granular | Active pharmaceutical ingredient | Brivanib alaninate | 30-80 | 40 - 70 |
| 2 | | Filler | Lactose monohydrate, microcrystalline cellulose | 15 - 65 | 25-55 |
| 3 | | Binder | Hydroxypropyl cellulose (HPC), polyvinyl pyrrolidone (PVP), starch, hydroxypropyl methylcellulose (HPMC) | 1 - 20 | 2 - 12 |
| 4 | | Disintegrant | Croscarmellose sodium, crospovidone, starch, sodium starch glycolate | 1 - 20 | 2 - 12 |
| 5 | Extra-granular | Filler | Lactose monohydrate, microcrystalline cellulose | 20-70 | 30-60 |
| 6 | | Disintegrant | Croscarmellose sodium, crospovidone, starch, sodium starch glycolate | 1 - 20 | 2 - 12 |
| 7 | | Lubricant | Magnesium stearate | 0.25-2.5 | 0.75 - 1.5 |

| | | | | | |
|---|---|---|---|---|---|
| * of levels that it may be used (% w/w of tablet weight) | | | | | |

**Table 3. Composition of brivanib alaninate tablet formulation used for the assessment of sticking tendency.**

| **S. No.** | **Location in tablet** | **Functional class** | **Examples of components** | **% w/w** |
|---|---|---|---|---|
| 1 | Intra-granular | Active pharmaceutical ingredient | Brivanib alaninate | 50 |
| 2 | | Filler | Microcrystalline cellulose | 24.5 |
| 3 | | Binder | Hydroxypropyl cellulose | 3 |
| 4 | | Disintegrant | Croscarmellose sodium | 3 |
| 5 | Extra-granular | Filler | Microcrystalline cellulose | 15 |
| 6 | | Disintegrant | Crospovidone | 3 |
| 7 | | Glidant | Colloidal silicon dioxide | 0.5 |
| 8 | | Lubricant | Magnesium stearate | 1 |

| | | | | |
|---|---|---|---|---|
| * of levels that it may be used (% w/w of core tablet weight) | | | | |

### Example 4 - Bioequivalence Study

A two-way crossover bioequivalence study was conducted in healthy volunteers to compare proposed commercial 400-mg high drug load (HDL) film-coated tablet manufactured by the wet granulation process with a 50% w/w drug load and two 200-mg low drug load (LDL) film-coated tablets manufactured by the roller compaction process with a 25% w/w drug load. Under fasted conditions, there were no significant differences between the pharmacokinetic (PK) profiles of the single oral dose 1 × 400-mg tablet of brivanib alaninate and the single oral dose 2 × 200-mg tablets of brivanib alaninate. The 90% CIs of the ratios of geometric least squares means and the potency-corrected geometric least squares means for PK parameters [AUC(0-T), AUC(INF), and Cmax] were entirely contained within 80% to 125%. These ratios are within the criterion 90% CI (80%-125%) for establishing bioequivalence. Therefore, the brivanib alaninate 1 × 400-mg tablet was bioequivalent to the brivanib alaninate 2 × 200-mg tablets in healthy subjects.

Although the foregoing invention has been described in some detail to facilitate understanding, it will be apparent that certain changes and modifications may be practiced within the scope of the appended claims. Accordingly, the described embodiments are to be considered as illustrative and not restrictive, and the invention is not to be limited to the details given herein, but may be modified within the scope and equivalents of the appended claims.

## Claims

1. A high drug load tablet formulation of Compound Ia of the formula 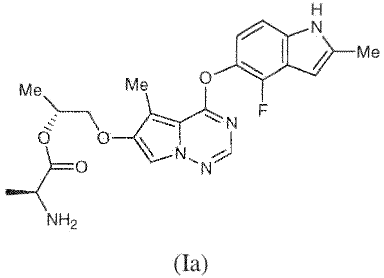 which comprises:
- Compound Ia as the active pharmaceutical ingredient in the range of 30-80% w/w,
- one or more fillers in the range of 15-65% w/w,
- binders in the range of 1-20% w/w,
- disintegrants in the range of 1-20% w/w, and
- lubricants in the range of 0.25-2.5% w/w,
wherein said formulation is obtainable by a wet granulation process.

2. The formulation of claim 1 wherein the active pharmaceutical ingredient is included in the range of 40-70% w/w, one or more fillers is included in the range of 25-55% w/w, the binder is included in the range of 2-12% w/w, one or more disintegrants is included in the range of 2-12% w/w, and the lubricant is included in the range of 0.75-1.5% w/w.

3. The formulation of any one of claims 1 or 2 wherein the active pharmaceutical ingredient is included in about 50% w/w, one or more fillers is included in about 24.5% w/w, the binder is included in about 3% w/w, one or more disintegrants is included in 3% w/w, the glidant is included in about 0.5% w/w and the lubricant is included in about 1.0% w/w.

4. The formulation of any one of claims 1 to 3 wherein the fillers and disintegrants are intragranular and extragranular.

5. The formulation of any one of claims 1 to 4 wherein the fillers are selected from lactose monohydrate and microcrystalline cellulose.

6. The formulation of any one of claims 1 to 5 wherein the binder is hydroxypropyl cellulose (HPC), polyvinyl pyrrolidone (PVP), starch or hydroxypropyl methylcellulose (HPMC).

7. The formulation of any one of claims 1 to 6 wherein the disintegrants are selected from Croscarmellose sodium, crospovidone, starch and sodium starch glycolate.

8. The formulation of any one of claims 1 to 7 wherein the lubricant is magnesium stearate.

9. The formulation of any one of claims 1 to 8 wherein the active pharmaceutical ingredient is brivanib alaninate, the fillers are microcrystalline cellulose, the binder is hydroxypropyl cellulose, the disintegrants are croscarmellose sodium and/or crospovidone, the glidant is colloidal silicon dioxide and the lubricant is magnesium stearate.

10. A formulation according to any one of claims 1 to 9 for use in a method of treating cancer.

11. A formulation for a use according to claim 10, wherein said cancer is selected from breast cancer, colorectal cancer, hepatocellular carcinoma, non-small cell lung cancer and prostate cancer.

12. A method for preparing the formulation according to any one of claims 1 to 9 using a wet granulation process.

## Patentansprüche

1. Zubereitungsform mit hoher Arzneimittelbeladung von Verbindung Ia der Formel 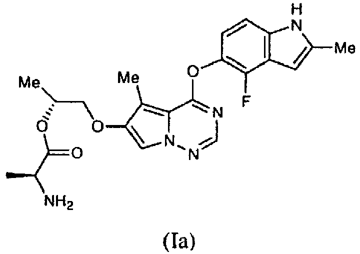 die umfasst:
- Verbindung Ia als den pharmazeutisch aktiven Bestandteil im Bereich von 30-80% Gewicht/Gewicht,
- einen oder mehrere Füllstoffe im Bereich von 15-65% Gewicht/Gewicht,
- Bindemittel im Bereich von 1-20% Gewicht/Gewicht,
- Zerfallhilfsmittel im Bereich von 1-20% Gewicht/Gewicht und
- Gleitmittel im Bereich von 0,25-2,5% Gewicht/Gewicht,
wobei die Zubereitung mit Hilfe eines Prozesses der Nassgranulierung erhalten werden kann.

2. Zubereitung nach Anspruch 1, wobei der pharmazeutisch aktive Bestandteil im Bereich von 40-70% Gewicht/Gewicht einbezogen ist, ein oder mehrere Füllstoffe im Bereich von 25-55% Gewicht/Gewicht einbezogen sind, das Bindemittel im Bereich von 2-12% Gewicht/Gewicht einbezogen ist, ein oder mehrere Zerfallhilfsmittel im Bereich von 2-12% Gewicht/Gewicht einbezogen sind und das Gleitmittel im Bereich von 0,75-1,5% Gewicht/Gewicht einbezogen ist.

3. Zubereitung nach einem der Ansprüche 1 oder 2, wobei der pharmazeutisch aktive Bestandteil in etwa 50% Gewicht/Gewicht einbezogen ist, ein oder mehrere Füllstoffe in etwa 24,5% Gewicht/Gewicht einbezogen ist, das Bindemittel in etwa 3% Gewicht/Gewicht einbezogen ist, ein oder mehrere Zerfallhilfsmittel in 3% Gewicht/Gewicht einbezogen sind, die Rieselhilfe in etwa 0,5% Gewicht/Gewicht einbezogen ist und das Gleitmittel in etwa 1,0% Gewicht/Gewicht einbezogen ist.

4. Zubereitung nach einem der Ansprüche 1 bis 3, wobei die Füllstoffe und Zerfallhilfsmittel intragranular und extragranular sind.

5. Zubereitung nach einem der Ansprüche 1 bis 4, wobei die Füllstoffe ausgewählt sind aus Lactose-monohydrat und mikrokristalliner Cellulose.

6. Zubereitung nach einem der Ansprüche 1 bis 5, wobei das Bindemittel Hydroxypropylcellulose (HPC), Polyvinylpyrrolidon (PVP), Stärke oder Hydroxypropylmethylcellulose (HPMC) ist.

7. Zubereitung nach einem der Ansprüche 1 bis 6, wobei die Zerfallhilfsmittel ausgewählt sind aus Natrium-Croscarmellose, Crospovidon, Stärke und Natrium-Stärkeglykolat.

8. Zubereitung nach einem der Ansprüche 1 bis 7, wobei das Gleitmittel Natriumstearat ist.

9. Zubereitung nach einem der Ansprüche 1 bis 8, wobei der pharmazeutisch aktive Bestandteil Brivanib alaninat ist, die Füllstoffe mikrokristalline Cellulose sind, das Bindemittel Hydroxypropylcellulose ist, die Zerfallhilfsmittel Natrium-Croscarmellose und/oder Crospovidon sind, das Rieselmittel kolloidales Siliciumdioxid ist und das Gleitmittel Magnesiumstearat ist.

10. Zubereitung nach einem der Ansprüche 1 bis 9 zur Verwendung in einer Methode zur Krebsbehandlung.

11. Zubereitung zur Verwendung nach Anspruch 10, wobei der Krebs ausgewählt ist aus Brustkrebs, Colorektalkrebs, hepatozellulärem Karzinom, nicht-kleinzelligem Lungenkrebs und Prostatakrebs.

12. Verfahren zum Herstellen der Zubereitung nach einem der Ansprüche 1 bis 9 unter Anwendung des Prozesses der Nassgranulierung.

## Revendications

1. Formulation de comprimé contenant une charge élevée, du Composé la de formule 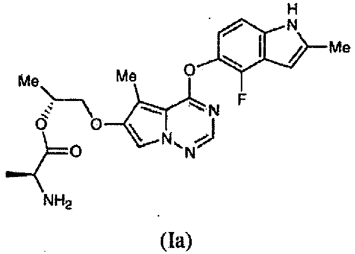 comprenant :
- le composé Ia en tant qu'ingrédient pharmaceutique actif dans la gamme de 30 à 80% p/p,
- une ou plusieurs charges dans la gamme de 15 à 65% p/p,
- des liants dans la gamme de 1 à 20% p/p,
- des désintégrants dans la gamme de 1 à 20% p/p, et
- des lubrifiants dans la gamme de 0,25 à 2,5% p/p,
ladite formulation pouvant être obtenue par un procédé de granulation par voie humide.

2. Formulation selon la revendication 1, dans laquelle l'ingrédient pharmaceutique actif est inclus dans la gamme de 40 à 70% p/p, une ou plusieurs charges est (sont) incluse(s) dans la gamme de 25 à 55% p/p, le liant est inclus dans la gamme de 2 à 12% p/p, un ou plusieurs désintégrants est (sont) inclus dans la gamme de 2 à 12% p/p et le lubrifiant est inclus dans la gamme de 0,75 à 1,5% p/p.

3. Formulation selon l'une quelconque des revendications 1 ou 2, dans laquelle l'ingrédient pharmaceutique actif est inclus à environ 50% p/p, une ou plusieurs charges est (sont) incluse(s) à environ 24,5% p/p, le liant est inclus à environ 3% p/p, un ou plusieurs désintégrants est (sont) inclus à 3% p/p, l'agent de glissement est inclus à environ 0,5% p/p et le lubrifiant est inclus à environ 1,0% p/p.

4. Formulation selon l'une quelconque des revendications 1 à 3, dans laquelle les charges et les désintégrants sont intragranulaires et extragranulaires.

5. Formulation selon l'une quelconque des revendications 1 à 4, dans laquelle les charges sont choisies parmi le lactose monohydraté et la cellulose microcristalline.

6. Formulation selon l'une quelconque des revendications 1 à 5, dans laquelle le liant est l'hydroxypropyl cellulose (HPC), une polyvinyl pyrrolidone (PVP), un amidon ou l'hydroxypropyl méthylcellulose (HPMC).

7. Formulation selon l'une quelconque des revendications 1 à 6, dans laquelle les désintégrants sont choisis parmi la croscarmellose sodique, la crospovidone, un amidon et le glycolate d'amidon sodique.

8. Formulation selon l'une quelconque des revendications 1 à 7, dans laquelle le lubrifiant est le stéarate de magnésium.

9. Formulation selon l'une quelconque des revendications 1 à 8, dans laquelle l'ingrédient pharmaceutique actif est le brivanib alaninate, les charges sont la cellulose microcristalline, le liant est l'hydroxypropyl cellulose, les désintégrants sont la croscarmellose sodique et/ou la crospovidone, l'agent de glissement est le dioxyde de silicium colloïdal et le lubrifiant est le stéarate de magnésium.

10. Formulation selon l'une quelconque des revendications 1 à 9, pour l'utilisation dans un procédé de traitement d'un cancer.

11. Formulation pour l'utilisation selon la revendication 10, dans laquelle ledit cancer est choisi parmi un cancer du sein, un cancer colorectal, un carcinome hépatocellulaire, un cancer du poumon non à petites cellules et un cancer de la prostate.

12. Procédé pour préparer Ia formulation selon l'une quelconque des revendications 1 à 9, en utilisant un procédé de granulation par voie humide.
